# EUROPEAN PATENT SPECIFICATION

(11) **EP 3 749 406 B1**
(45) Date of publication and mention of the grant of the patent: **24.07.2024**
(21) Application number: 19750916.9
(22) Date of filing: 08.02.2019
(51) Int. Cl.: A61M 25/10, A61B 17/24, A61B 17/28, A61B 17/29, A61B 17/34, A61M 25/00, A61M 29/02, A61B 17/32

(54) **SUBMUCOSAL DRUG DELIVERY APPARATUSES AND SYSTEMS**
VORRICHTUNGEN UND SYSTEME ZUR SUBMUKOSALEN ARZNEIMITTELVERABREICHUNG
APPAREILS ET SYSTÈMES D'ADMINISTRATION SOUS-MUCOSIQUE DE MÉDICAMENT

(30) Priority: 08.02.2018 US 201862627878 P
(43) Date of publication of application: 16.12.2020
(73) Proprietor: Massachusetts Eye and Ear Infirmary, Boston, Massachusetts 02114 (US)
(72) Inventor: BLEIER, Benjamin S., Weston, MA 02493 (US)
(74) Representative: Forresters IP LLP
(86) International application number: PCT/US2019/017178
(87) International publication number: WO 2019/157259

(56) References cited:
- CN-A- 107 898 540
- US-A1- 2005 124 977
- US-A1- 2005 240 147
- US-A1- 2013 085 472
- US-A1- 2013 174 849
- US-A1- 2014 257 237
- US-A1- 2015 133 851
- US-A1- 2015 165 176
- US-A1- 2015 320 972
- US-A1- 2016 193 145
- US-A1- 2017 150 984

## Description

### RELATED APPLICATIONS

The present application claims priority to U.S. Provisional Patent Application No. 62/627,878, filed February 8, 2018, entitled, "SUBMUCOSAL DRUG DELIVERY APPARATUSES, SYSTEMS, AND METHODS,".

### TECHNICAL FIELD

The present application relates generally to the field of drug delivery.

### BACKGROUND

Various neurological disorders can be treated through the delivery of drugs or therapeutic agents to the central nervous system. Biological defenses such as the blood brain-barrier prevent many therapeutic agents, such as proteins and oligonucleotides, from reaching the central nervous system, thereby inhibiting the efficacy of the therapeutic agents. Surgically implanted mucosal grafts allow therapeutic agents to be effectively delivered at high molecular weight to the central nervous systems; however, certain patients, such as infant and pediatric patients, are not well suited for surgical treatment.

Direct transnasal delivery (e.g., spaying) of therapeutic agents for diffusion through the olfactory mucosa is one non-surgical method for delivering therapeutic agents to patients not well suited for or surgery or for whom surgery is impractical or impossible. However, direct transnasal delivery has highly inefficient and variable drug distribution and drug residence time in view of the variation and limitations of available surface area for delivery to the olfactory through topical nasal applications.

US 2005/0240147 A1 discloses a nasal drug delivery device having a catheter with a balloon.

### SUMMARY

The invention is defined by claim 1. Further embodiments of the invention are defined by the dependent claims. Methods as such are not part of the claimed invention.

The inventor has discovered that the efficacy of non-invasive delivery of therapeutic agents to the central nervous system is greatly enhanced through controlled depot delivery directly to the submucosal space of the olfactory mucosa. The controlled depot delivery elutes drug over a prescribed period of time based on the polymer or carrier used. By dosing in the submucosal space, the barriers to diffusion to the olfactory nerves are minimized relative to transepithelial delivery and the drugs are thereby protected from multiple degradative enzymes present in nasal mucus.

Accordingly, various embodiments disclosed herein provide apparatuses, systems, and example methods for a controlled depot delivery where a drug can be delivered directly to the submucosal space of the olfactory mucosa. The apparatuses create a precision controlled pocket in this space in a reproducible manner to standardize the volume of drug delivery. The apparatuses permit delivery of an array of drug carriers from solutions and thermosensitive polymers to solid formulations.

Various embodiments provide drug delivery devices for submucosal drug delivery. The drug delivery devices include a catheter component comprising a stem portion and a branch portion. The catheter component includes a pre-defined bend whereby the branch portion is angled with respect to the stem portion at an obtuse angle. The branch portion includes a dissecting head and at least one delivery port. The dilation balloon is positioned on the branch portion between the pre-defined bend and the dissecting head. The dilation balloon can advance from the outer sheath along the branch portion. Once the dilation balloon advances into position, the dilation balloon is configured to expand around at least a portion of a cylindrical wall of the branch portion. The drug delivery device includes a drug delivery reservoir fluidly coupled to the drug delivery port through the branch portion and through at least a portion of the stem portion. The drug delivery device includes a handle portion coupled to the stem portion.

The dilation balloon enables pocket formation to precise specifications through a small mucosal puncture, thereby preventing loss of drug through the mucosal puncture site and minimizing trauma that can occur to the mucosa over repeated injections.

In certain implementations, the drug delivery device includes an outer sheath positioned between the pre-defined bend and the dilation balloon.

In certain implementations, the branch portion is configured to retract into and extend from the outer sheath at the obtuse angle.

In certain implementations, at least a portion of the branch portion is configured to retract into and extend from the outer sheath at the obtuse angle.

In certain implementations, the dilation balloon is configured to retract into and extend from the outer sheath at the obtuse angle with the branch portion.

In some implementations, the devices also enable direct visualization of the pocket to enable precise pocket placement in the correct plane while minimizing the risk of injury or penetration of the overlying mucosa and prevent injury or penetration of the skull base. Accordingly, certain implementations comprise an onboard micro-endoscope device that enables direct visualization and pocket development during navigation of the narrow olfactory cleft by the drug delivery device. The endoscope is positioned adjacent to the pre-defined bend so as to have a field of view of the dissecting head and the plane above the dissecting head.

In some implementations, the endoscope comprises a camera.

In certain implementations, the dissecting head comprises a flattened disc geometry.

In certain implementations, the dissecting head comprises a beveled edge.

In certain implementations, the dissecting head comprises a cannula having a diameter in the range of 0.25 mm and 3 mm.

In certain implementations, the drug delivery device includes a pump communicably coupled to the drug delivery reservoir and configured to move a drug from the drug delivery reservoir to the at least one delivery port.

In certain implementations, the pump comprises a plunger assembly.

In certain implementations, the obtuse angle is in the range of 155-170 degrees.

In certain implementations, the branch portion has a length in the range of 10-20cm.

In certain implementations, the balloon has length in the range of 1-4 cm.

In certain implementations, the balloon has a diameter in the range of 0.1-3 mm.

In certain implementations, the balloon is configured to dilate asymmetrically.

In certain implementations, the branch is configured to extend 2-3 cm pass the outer sheath.

In certain implementations, the branch portion extends upstream and downstream of the balloon portion such that the balloon is positioned between the dissecting head a section of the branch portion that is narrower than balloon in an inflated state, whereby the cross section of the entry to the depot upon retraction of the branch portion is narrower than the cross section of the depot.

In certain implementations, the reservoir contains a therapeutically effective amount of a drug for treating a central nervous system disorder. In certain implementations, the therapeutically effective amount of the drug from treating the central nervous system disorder comprises at least one of oligonucleotides and antibodies. In certain implementations, the therapeutically effective amount of the drug from treating the central nervous system disorder comprises at least one pharmaceutical agent with a molecular size of greater than 500 Da (e.g., greater than 600 Da, 700 Da, 800 Da, 900 Da, 1 kDa, 2 kDa, 3 kDa, 4 kDa, 5 kDa, 10 kDa, 20 kDa, 30 kDa, 70 kD, or 100 kD) or a net positive or net negative charge. In certain implementations, the therapeutically effective amount of the drug from treating the central nervous system disorder comprises at least one pharmaceutical agent that is a polar molecule. In certain implementations, the therapeutically effective amount of the drug comprises at least one pharmaceutical agent present in the composition that decreases unregulated or mis-regulated cell growth (e.g., reduces the rate of cancer cell growth), mediates or induces cancer cell death (e.g., necrosis or apoptosis), decreases protein misfolding and/or aggregation, mediates an increase or decrease in neurohormone or neurotransmitter production or turn-over, decreases loss of myelin, reduces neuronal cell death or neuronal loss, reduces loss of axons, mediates an increase or decrease in neurohormone or neurotransmitter receptor activity, mediates an increase or decrease in synaptic transmission between neurons, and mediates an increase or decrease in neuronal intracellular signaling pathways. In some compositions, the at least pharmaceutical agent is an analgesic.

In certain implementations, the therapeutically effective amount of the drug comprises at least one of a chemotherapeutic agent, L-DOPA, carbidopa, an anti-depressant agent, an anti-psychotic agent, donepezil, rivastigmine tartrate, galantamine, memantine, ISIS-SOD1, ISIS-SMN, ISIS-TTR, ELND005, β- or γ-sectretase inhibitors, neurotrophic peptides, nanoparticles, fusion proteins, and gene therapy vectors.

Non-limiting examples of chemotherapeutic agents include proteins (e.g., antibodies, antigen-binding fragments of antibodies, or conjugates or derivatives thereof), nucleic acids, lipids, or small molecules, or combinations thereof. Non-limiting examples of chemotherapeutic agents include: cyclophosphamide, mechlorethamine, chlorabucil, melphalan, daunorubicin, doxorubicin, epirubicin, idarubicin, mitoxantrone, valrubicin, paclitaxel, docetaxel, etoposide, teniposide, tafluposide, azacitidine, azathioprine, capecitabine, cytarabine, doxifluridine, fluorouracil, gemcitabine, mercaptopurine, methotrexate, tioguanine, bleomycin, carboplatin, cisplatin, oxaliplatin, all-trans retinoic acid, vinblastine, vincristine, vindesine, vinorelbine, and bevacizumab (or an antigen-binding fragment thereof). Additional examples of chemotherapeutic agents are known in the art.

Non-limiting examples of anti-depressant agents include: selective serotonin reuptake inhibitors (e.g., citalopram, escitalopram, fluoxetine, paroxetine, or sertraline), serotonin-norepinephrine reuptake inhibitors (e.g., desvenlafaxine, duloxetine, milnacipran, and venlafaxine), noradrenergic and specific serotonergic antidepressants (e.g., mianserin and mirtazapine), norephinephrine reuptake inhibitors (e.g., atomoxetine, mazindol, reboxetine, and viloxazine), norepinephrine-dopamine reuptake inhibitors (e.g., bupropion), selective serotonin reuptake enhancers (e.g., tianeptine), norephinephrine-dopamine disinhibitors (e.g., agomelatine), tricyclic antidepressants (e.g., amitriptyline, clomipramine, doxepin, imipramine, and trimipramine), secondary amine tricyclic depressants (e.g., desipramine, nortriptyline, and protripyline), monoamine oxidase inhbitors (e.g., isocarboxazid, moclobemide, phenelzine, selegiline, and tranylcypromine), buspirone, gepirone, nefazodone, trandospirone, trazodone, bupropion, benzodiazepines, amphetamine, methylphenidate, modafinil, lithium, carbamazepine, sodium valproate, and lamotrigine. Non-limiting examples of anti-psychotic agents include risperidone, olanzapine, and quetiapine.

In certain implementations, the dissecting head is configured to create an opening having a cross sectional area that is smaller than a cross sectional area of the dilation balloon when inflated.

Various implementations provide example methods of drug delivery. The methods include inserting a catheter component of a drug delivery device into a submucosal tissue by penetrating the tissue via a dissecting head positioned at an end of a branch portion of the catheter component extending from a stem portion of the catheter component. The catheter component includes a pre-defined bend whereby the branch portion is angled with respect to the stem portion at an obtuse angle. The methods include creating a depot in the submucosal tissue by inflating a dilation balloon positioned on the branch portion between the pre-defined bend and the dissecting head to compress the submucosal tissue. The methods include deflating the dilation balloon. The methods include ejecting a drug through a delivery port in the dissecting head. The drug is pushed or pumped from a drug reservoir through the stem portion and the branch portion.

All combinations of the foregoing concepts and additional concepts discussed in greater detail below (provided such concepts are not mutually inconsistent) are part of the subject matter disclosed herein. In particular, all combinations of claimed subject matter appearing at the end of this disclosure are part of the inventive subject matter disclosed herein.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

Other features and advantages will be apparent from the following detailed description, and from the claims.

### BRIEF DESCRIPTION OF THE DRAWINGS

The skilled artisan will understand that the drawings primarily are for illustrative purposes and are not intended to limit the scope of the inventive subject matter described herein. The drawings are not necessarily to scale; in some instances, various aspects of the inventive subject matter disclosed herein may be shown exaggerated or enlarged in the drawings to facilitate an understanding of different features. In the drawings, like reference characters generally refer to like features (e.g., functionally similar and/or structurally similar elements).
FIG. 1 shows a top perspective view of a drug delivery device as described herein.
FIG. 2 illustrates a front sectional view of the drug delivery device of FIG 1.
FIGS. 3A-3B show a front sectional anatomical view magnifying the submucosal, supramucoperiosteal space of the olfactory mucosa in which a drug is delivered using drug delivery devices as described herein.
FIGS. 4A-4P illustrate a method for submucosal drug delivery via the drug delivery device of FIG. 1.
FIGS. 5A-5B illustrate a drug delivery device according to particular implementations.
FIG 6 shows a side sectional view of another drug delivery device according to certain implementations.
FIG. 7 illustrates a cross sectional illustration of a rat model of a depot.
FIG. 8 shows an endoscopic view of the olfactory submucosal space with an olfactory nerve trunk (labeled) seen extending from the intracranial space into the submucosal space.
FIG. 9 is a cross sectional histological view (H&E) of the rat model depicted in FIG. 7 at low and high magnification.
FIG. 10 shows a comparison of ipsi-lateral brain-derive neurotrophic factor, BDNF, upregulation (e.g. craniotomy side) following BDNF AntagoNAT (0. 15mg/kg) delivery.
FIG. 11 shows a comparison of contra-lateral BDNF upregulation (e.g. non-craniotomy side) following BDNF AntagoNAT (0. 15mg/kg) delivery.
FIG. 12 shows a comparison of, BDNF, AntagoNAT (0. 15mg/kg, 60micL) distribution in the rat brain following depot delivery versus traditional nasal irrigation and negative saline control.

The features and advantages of the inventive concepts disclosed herein will become more apparent from the detailed description set forth below when taken in conjunction with the drawings.

### DETAILED DESCRIPTION

Following below are more detailed descriptions of various concepts related to, and exemplary embodiments of, inventive systems, methods, and components related to optical fiber coupling systems.

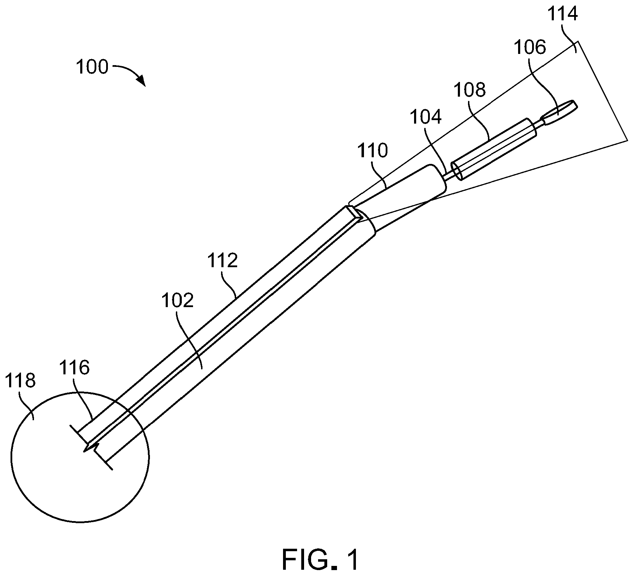

FIG. 1 shows a top perspective view of a drug delivery device as described herein. A drug delivery device 100 is particularly suited for olfactory submucosal depot delivery of a drug or therapeutic agent. As described in further detail herein, the drug delivery device 100 allows a large standardized space to be created with a pinhole opening to enable a precise volume of a therapeutic agent to be deposited trans olfactory while substantially preventing the drug from leaking out of the submucosal space. The drug delivery device 100 includes a catheter component 100 having two portions, a stem portion 102 and a branch portion 104. The catheter component 100 includes a pre-defined bend about a point so that the branch portion 104 of the catheter component 100 is positioned at an angle with respect to the stem portion 102. In particular, the branch portion 104 is positioned at an obtuse angle with respect to the stem portion 102. In certain implementations, the branch portion 104 is angulated with respect to the stem portion 102 in the range of 155-170 degrees (i.e. the angle between the stem portion 102 and the branch portion 104). The catheter component 100 is composed out of surgical metal in certain implementations and has a rigidity that allows it to maintain the pre-defined angulated shape during insertion. The angulation of the branch portion 104 with respect to the stem portion 102 allows the drug delivery depot or space to be properly created in the olfactory mucosa while limiting or precluding intracranial penetration or penetration of the skull base. The angulation of the branch portion 104 with respect to the stem portion 102 allows the drug delivery depot to be created substantially parallel to the olfactory bulb in the submucosal space, for example in supramucoperichondrial or submucoperichondrial plane.

The catheter component 100 includes a dissecting head 106 positioned at the tip of the branch portion 104. The dissecting head 106 is on a leading edge of the branch portion 104 to create a pinhole opening through the olfactory mucosa tissue as the drug delivery device is advanced therein. The dissecting head 106 includes a flattened disc geometry (e.g., flat semi-circular) in accordance with the present claimed invention. The dissecting head 106 includes a tapered or beveled geometry in certain implementations. The dissecting head comprises a port coupled via a channel extending through the branch portion 104 and at least a portion of the stem portion 102. Accordingly, the dissecting head is configured as a cannula or a needle tip in certain implementations. The port provides a pathway for fluids to be delivered through the catheter component 100 to the depot created thereby. In certain implementations, the port is used to retrieve anatomical fluids. The port is connected to a reservoir 118 in certain implementations. The reservoir 118 can be positioned adjacent to and/or in a handle portion 116. The reservoir 118 is used to hold a drug or the therapeutic agent for delivery. The drug can be pumped from the reservoir 118 to the port via an actuator generating a change in pressure in the catheter component 100. The drug can include a liquid, a gel (e.g., thermosensitive gels), and/or a solid in accordance with certain implementations.

The catheter component 100 also includes a dilation balloon 108, positioned on the branch portion. The dilation balloon 108 is positioned between the pre-defined bend in the catheter component and the dissecting head 106. The dilation balloon 108 is configured to expand upon actuation. The actuation can be initiated via an actuator button positioned on or near the handle 116. In some implementations, the dilation balloon is fluidly coupled to a fluid source for increasing the volume of the balloon. Expansion of the dilation balloon creates the olfactory submucosal depot by compressing the tissue in the region. The dilation balloon 108 is positioned so that the dissecting head 106 is upstream of the dilation balloon 108 and at least a portion of the branch portion 104 is downstream of the dilation balloon 108. This configuration ensures that once the dilation balloon 108 is contracted back from the expanded state, before the branch portion is at least partially retracted, the pinhole opening created via the dissecting head 106 upon entry into submucosa space maintains its size (e.g. smaller than the depot and smaller than the expanded dilation balloon). In certain implementations, the dilation balloon 108 has a length in the range of 1-4 cm, an inflated diameter in the range of 0.1-3mm. The dilation balloon 108 is symmetrical in certain implementations, but may also be configured asymmetrically in particular implementations. Maintaining the size of the opening smaller than the depot creates a bottleneck to limit the flow or exit of any delivered drug from the opening through which the dissecting head 106 created and is extracted. As discussed further herein, after the dilation balloon is contracted from its expanded state, the branch portion 104 is at least partially retracted from the depot or void created by the dilation balloon 108 and the drug or therapeutic agent is ejected through the port in the branch portion to reside in the depot for diffusion into the central nervous system. While the dilation balloon 108 is illustrated by way of example as cylindrical, the dilation balloon can be formed in a variety of different shapes or geometries to form various depot geometries and size, for example depending on the patient, dosage, and/or therapeutic agent or drug.

In certain implementations, the catheter component 100 also includes an outer sheath 110. The outer sheath 110 is positioned downstream of the bend in the catheter component 100. The outer sheath 110 is configured to house the branch portion 104 in implementations where the branch portion 104 is partially or wholly retractable into the outer sheath 110. The outer sheath 110, is also angulated with respect to the stem portion 102 in the range of 155-170 degrees. The catheter component 100 can also include an endoscopic device 112 positioned on the stem portion. The endoscopic device 112 is positioned adjacent the pre-defined bend so as to have a field of view of the dissecting head 106 and the plane directly above the dissecting head 106. This assists the operator with properly placing the dissecting head 106 and the dilation balloon 108 in the submucosal tissue. The endoscopic device 112 can include a camera (e.g. a digital video camera) for recording the views obtainable via the endoscopic device 112.

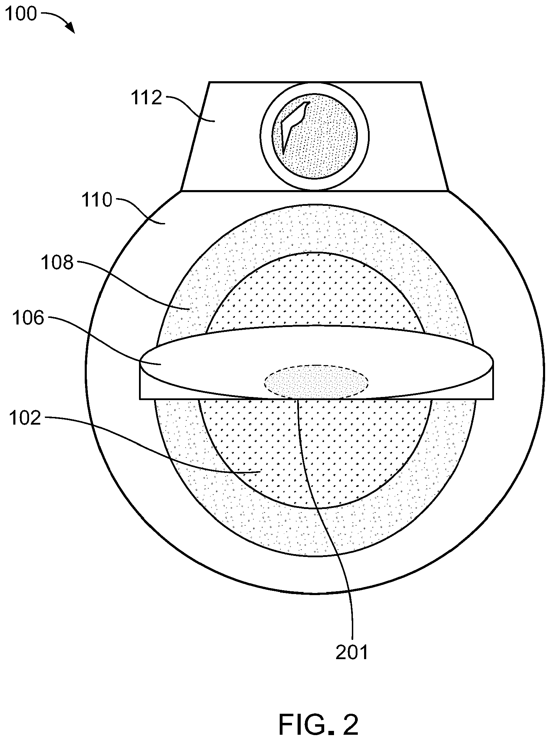

FIG. 2 illustrates a front sectional view of the drug delivery device of FIG 1. The drug delivery port 201 positioned in the dissecting head 106 of the branch portion 104 is visible in FIG. 2. As shown in FIG. 2, the dissecting head 106 is sized to create an opening sufficient for the branch portion 104 to extend through, but smaller than the outer sheath 110 into which the balloon 108 can be retracted. In certain implementations, the branch portion 104 has a length in the range of 2-4 cm and a diameter in the range of. In certain implementations, the outer sheath 110 has a length in the range of 8-15 cm and a diameter in the range of 2-3 mm. The optional endoscope 112 is positioned atop the stem portion 102 to provide a view of the position of the dissecting head 106 and the dilation balloon 108. The camera of the endoscope 112 allows the operator to watch the surface of the mucosa so that the operator can ensure that as the dissecting head 106 and the branch portion 104 are advanced, they are advanced backwards rather than upwards. The camera allows the operator to watch the area/plane above the dissecting head 106, while the dissecting head and dilation balloon are operating in the plane below.

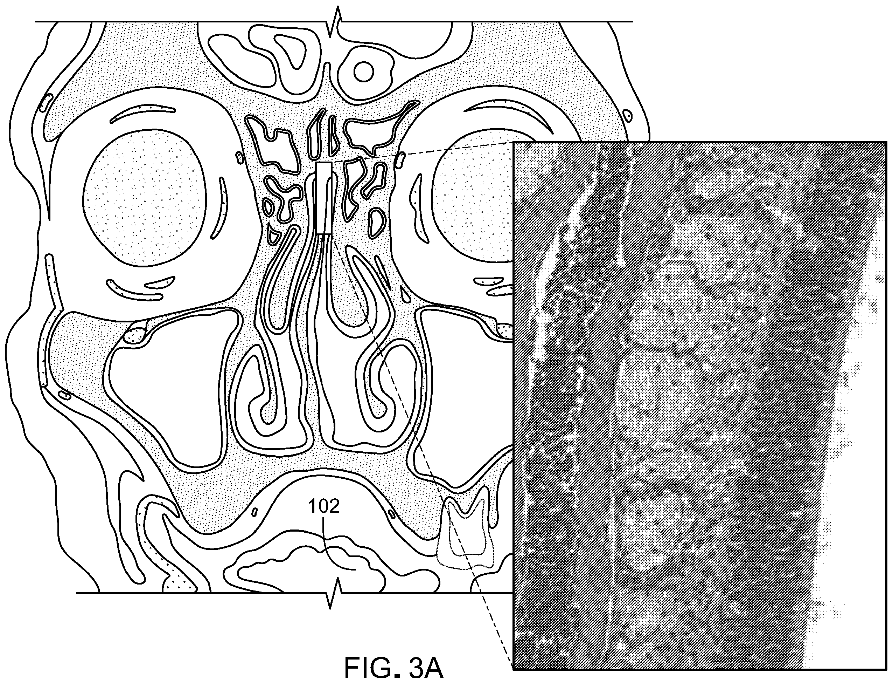

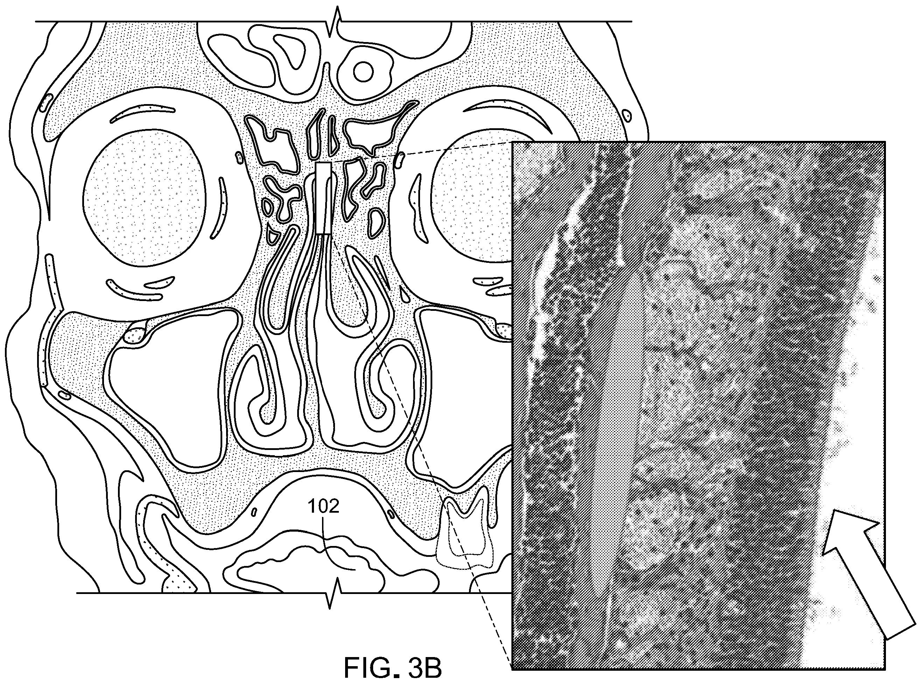

FIGS. 3A-3B show a front sectional anatomical view magnifying the submucosal, space of the olfactory mucosa in which a drug is delivered using a drug delivery device as described herein. In particular, FIG. 3A shows a magnified view of submucosal layers in which the depot for drug delivery will be created and FIG. 3B shows the depot 302 created in the mucosal layers after the depot is generated.

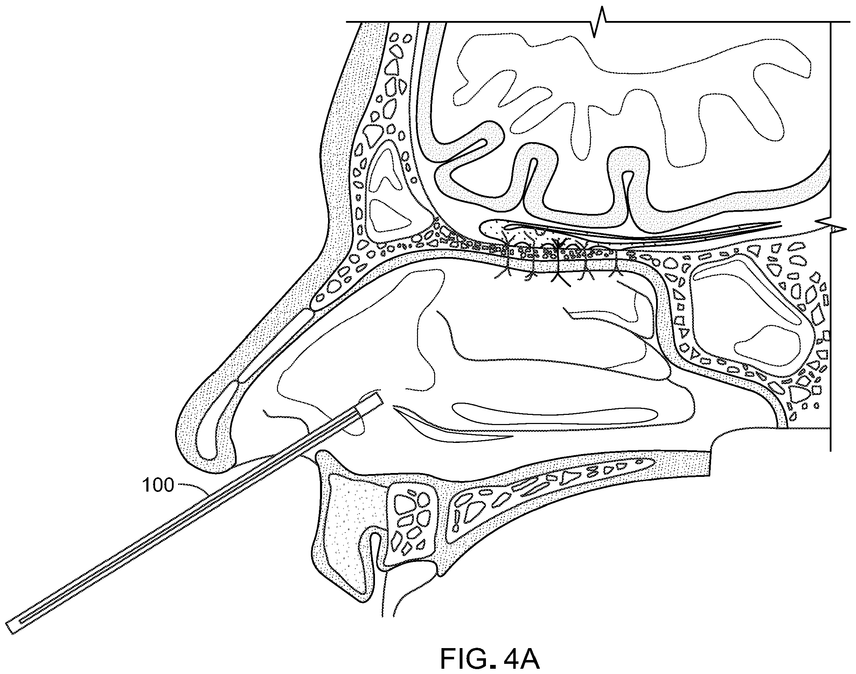

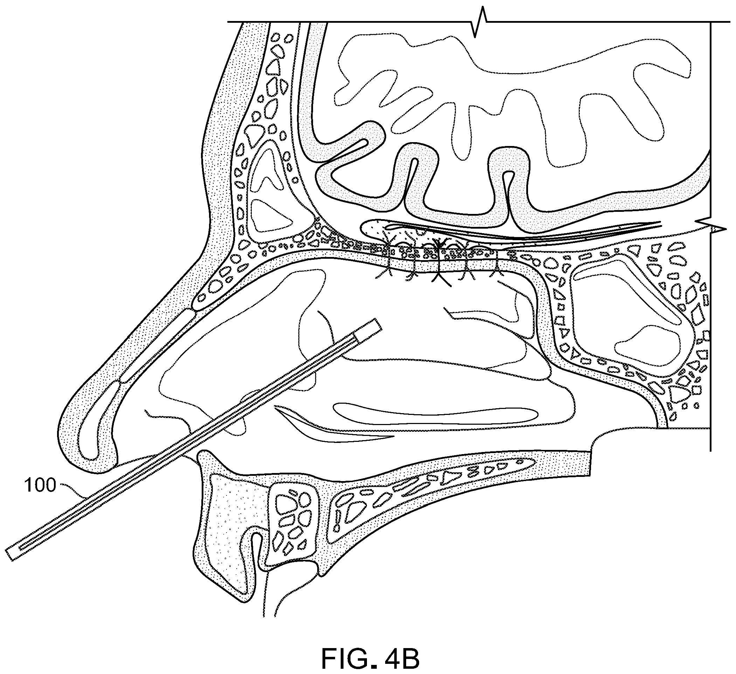

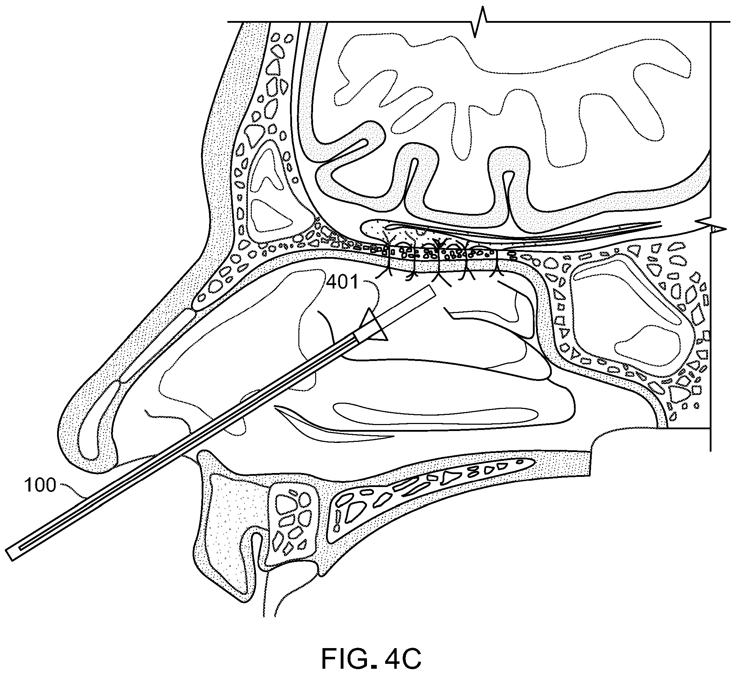

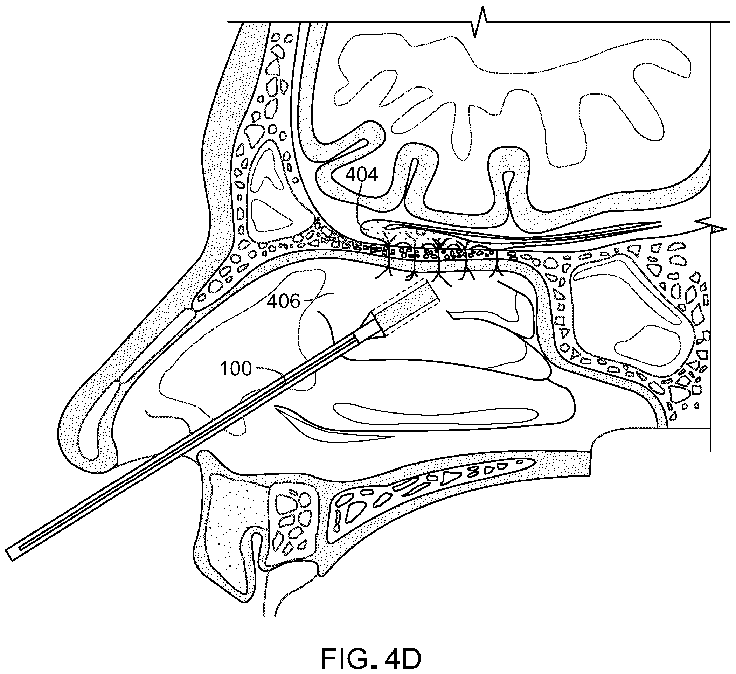

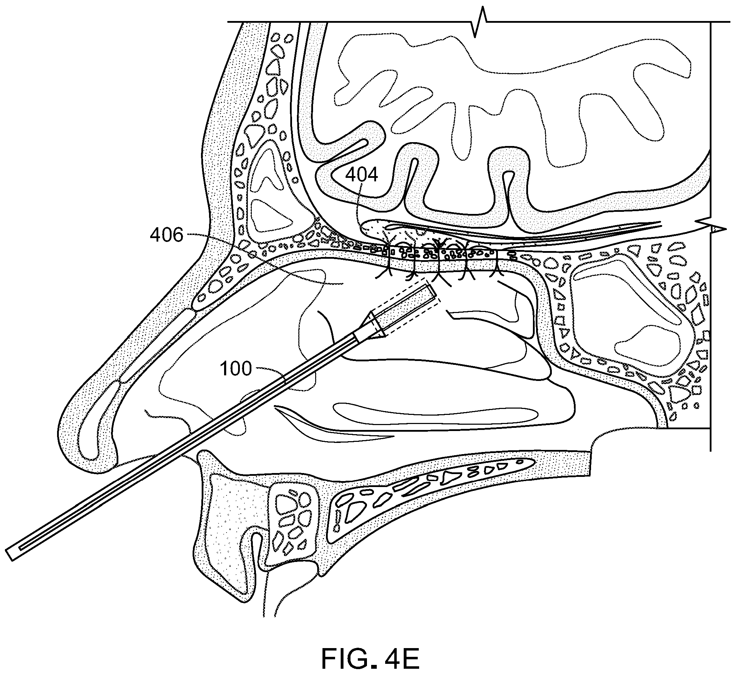

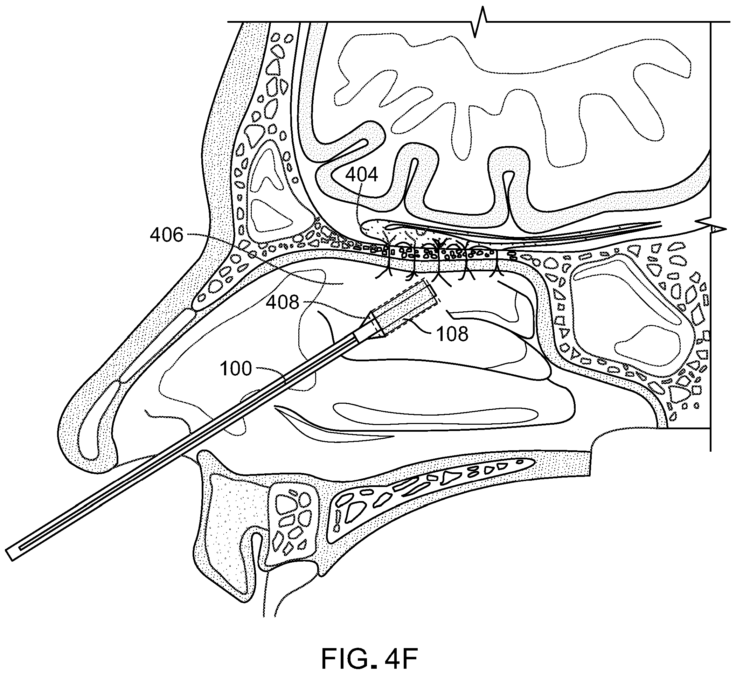

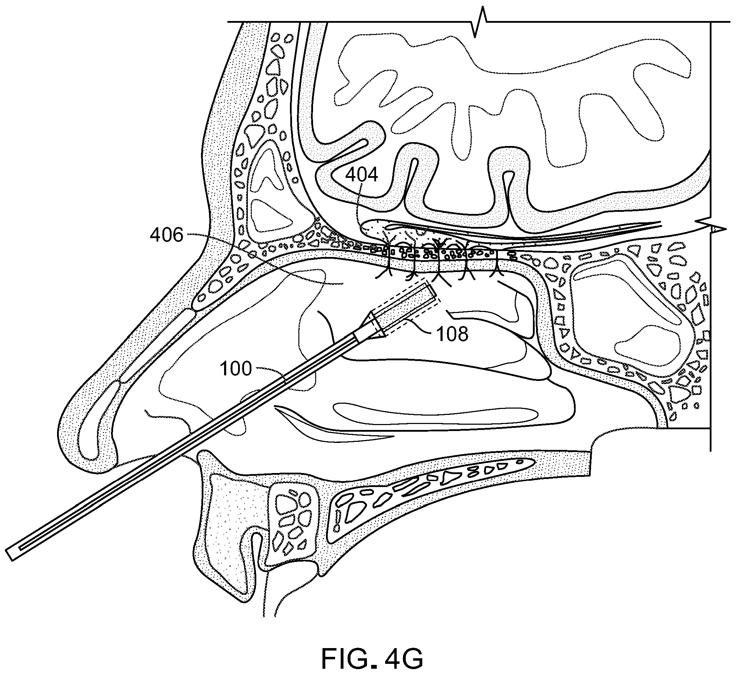

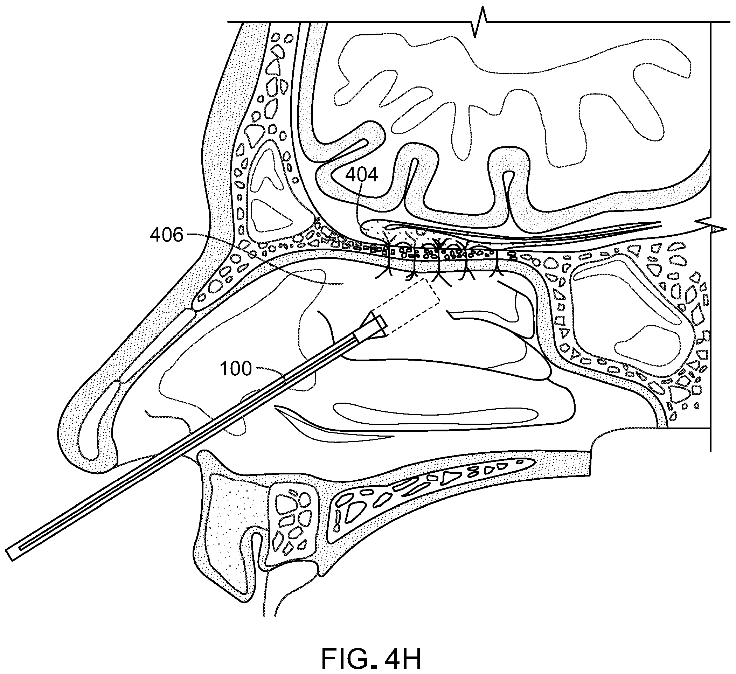

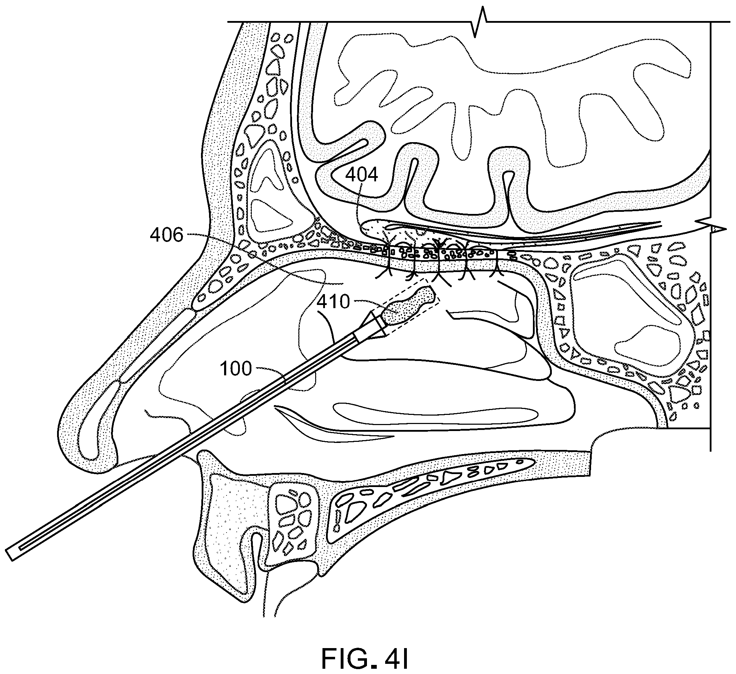

FIGS. 4A-4L illustrate a method for submucosal drug delivery via the drug delivery device of FIG. 1. In FIG. 4A, the drug delivery device 100 is inserted into the nasal cavity. In retractable configurations, the dissecting head 106 can be retracted into the outer sheath until device 100 encounters tissue to be penetrated. As shown in FIG. 4B, the drug delivery device penetrates the submucosal tissue. The dissecting head 106 is extended from the outer sheath 110 at the predefined bend angle. As demonstrated in FIG. 1, the outer sheath 110 and the branch portion are at the predefined bend angle in both the extended and the retracted position. The endoscope 110 is used for guidance during penetration, and the dissecting head 106 generates the pinhole opening through which the branch portion 104 extends. FIG. 4C shows the delivery device 100, with the branch portion 104 extending through the opening 401 created by the dissecting head 106. FIG. 4D shows the delivery device 100 with the dilation balloon 108 beginning to deploy. As shown in FIG. 4E, the branch portion 104 of the device 100 is substantially parallel to the olfactory bulb 404 and positioned in the submucosal space 406. In FIG. 4F, the dilation balloon 108 is fully deployed and thereby pushes the mucosal tissue apart to create a depot or void 408 within the tissue to be filled by the drug or therapeutic agent. In FIG. 4G, the dilation balloon 108 is contracted leaving a space in the submucosal tissue into which the therapeutic agent will be delivered. In FIG. 4H, the delivery device is retracting. During the retraction of the delivery device, for example, the branch portion 104 can be retracted at least in part into the outer sheath 110. The branch portion can be retracted such that the balloon is inside the outer sheath 110 while the dissecting head is still extending from the outer sheath and is positioned inside the depot 408 with the delivery port 201 positioned in the depot 408 or at the narrowed opening entry way into the depot 408. The outer sheath 110 may not be extended through the opening created by the dissecting head 106 in order to minimize the size of the opening and prevent the flow of the drug or therapeutic agent from the depot 408. In FIG. 4I, the drug or therapeutic agent 410 is ejected from device 100 through the delivery port 201. The therapeutic agent 410 can be pumped from a reservoir 118 in or near the handle 116 through the stem portion 102 and through the branch portion 104 (e.g. via a channel or conduit extending contiguously through the stem portion 102 and 104). Accordingly, the system is operable via single-handed operation to penetrate, space, and inject. The therapeutic agent 410 can also be contained in vial or cartridge that can be coupled to device 100 and pumped from the cartridge through the stem and branch portions 102, 104 respectively and out of the delivery port 201. In certain implementations, the device 100 includes two plungers in the handle a first plunger for inflating and deflating the dilation balloon and a second plunger for injecting the therapeutic agent 410. For example, the device 100 can be configured as a syringe.

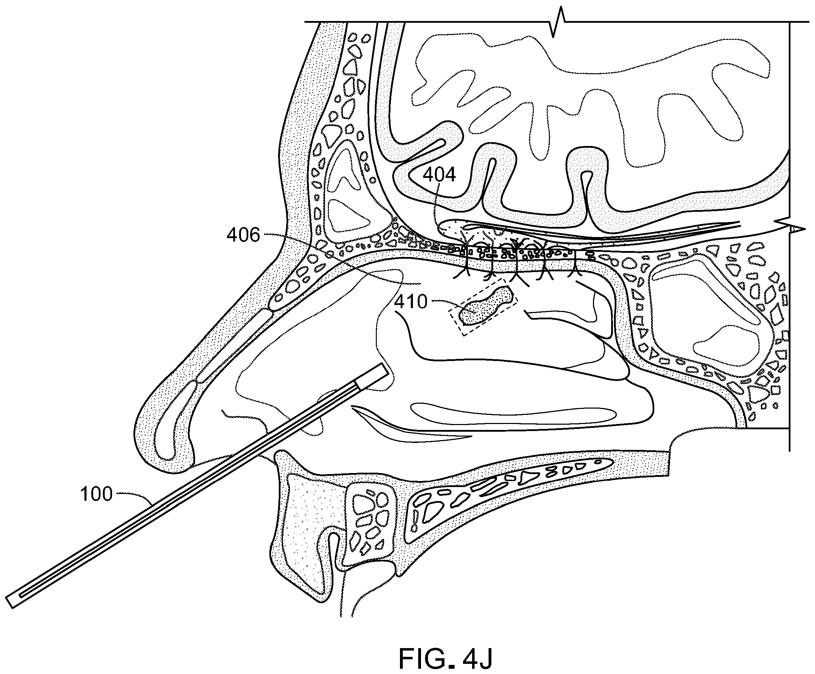

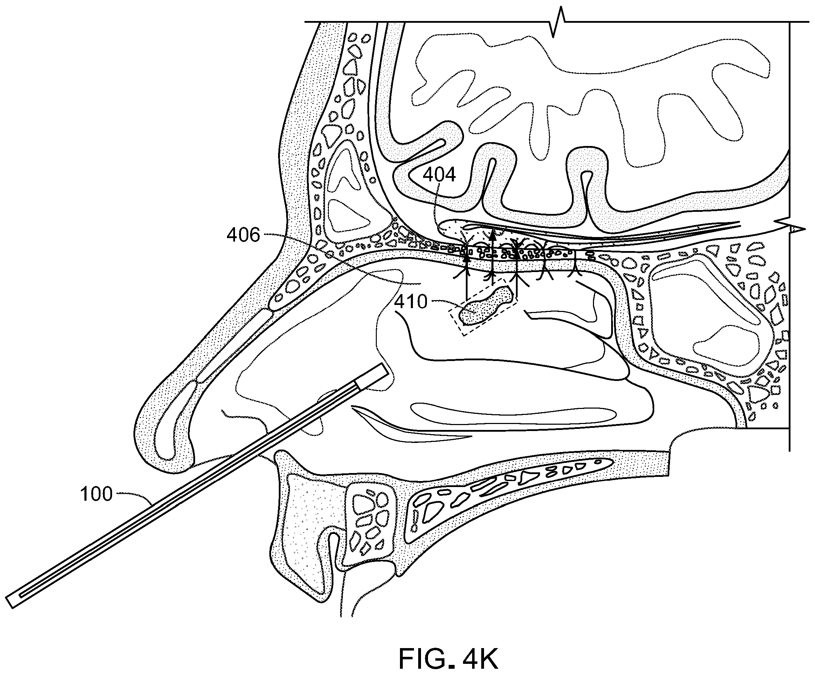

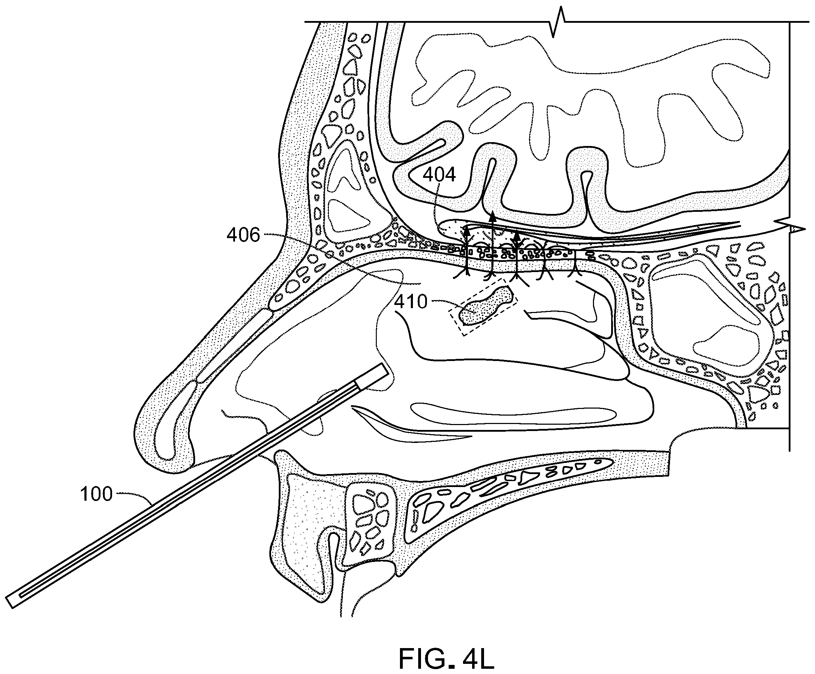

In FIG. 4J the device 100 is substantially removed from the depot 408, and in FIG. 4K the therapeutic agent 410 is diffused into the central nervous system via the olfactory bulb 404. In FIG. 4L, the agent 410 retained in the depot 408 is reduced further as uptake into the central nervous system increases over time. Accordingly, embodiments of the present invention provide an alternative method of treating conditions affecting the central nervous system that presently warrant general anesthesia, surgery, and/or intrathecal injections. The devices and methods afforded by implementations disclosed herein limit drug leak out, thereby increasing the efficacy and allowing a precise amount of a drug or therapeutic agent to be administered.

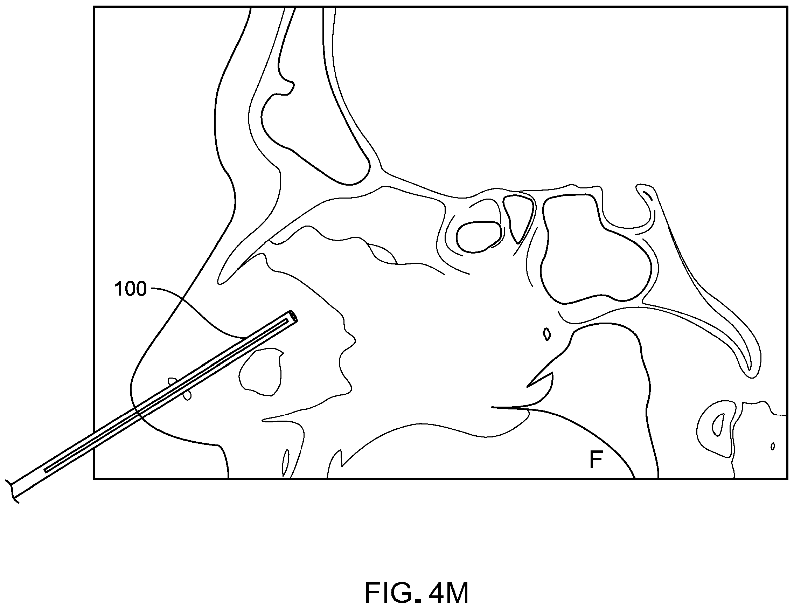

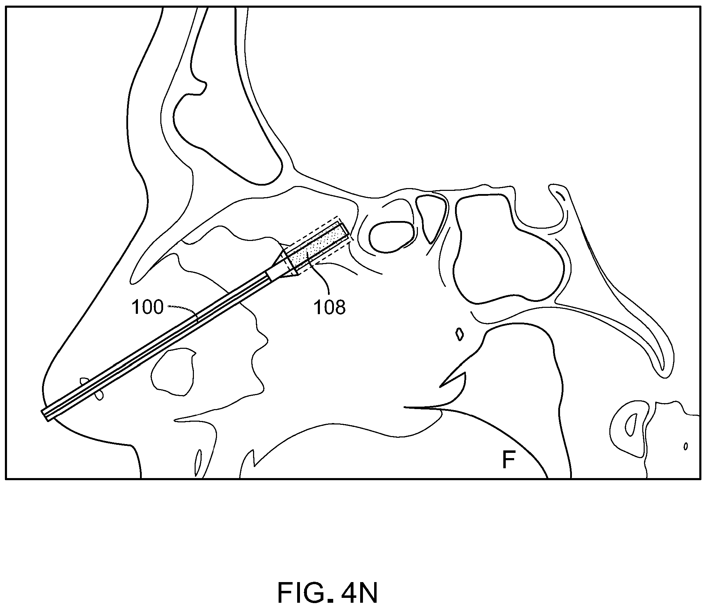

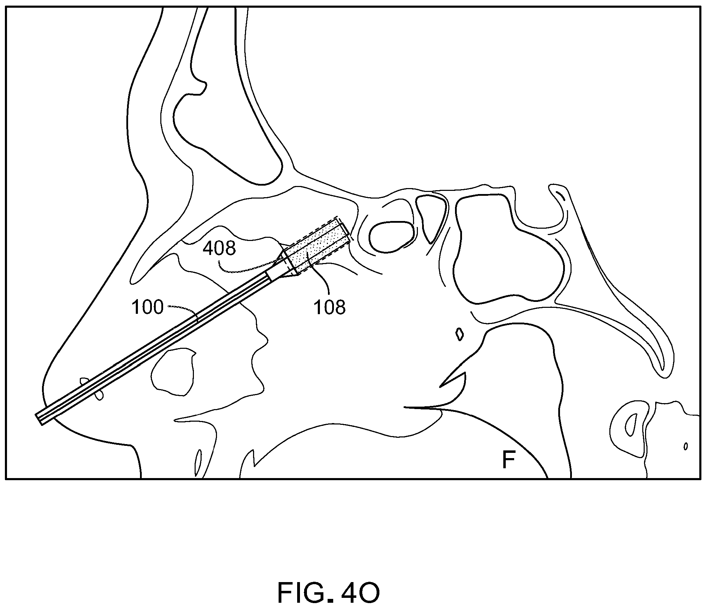

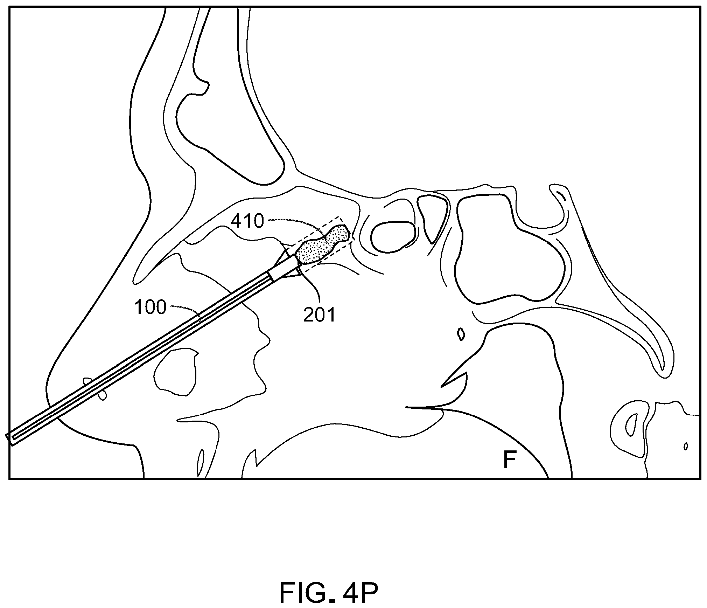

FIGS. 4M-4P illustrate the method for submucosal drug delivery of FIGS. 4A-4L depicted by a CT scan along the septum. In FIG. 4M, the drug delivery device 100 is inserted into the nasal cavity. In FIG. 4N, the dilation balloon 108 of the delivery device 100 is beginning to deploy. In FIG. 4O, the dilation balloon 108 is fully deployed and thereby pushes the mucosal tissue apart to create a depot or void 408 within the tissue to be filled by the drug or therapeutic agent. In FIG. 4P, the delivery device 100 is partially retracted (e.g. into the outer sheath) and the drug or therapeutic agent 410 is ejected from device 100 through the delivery port 201.

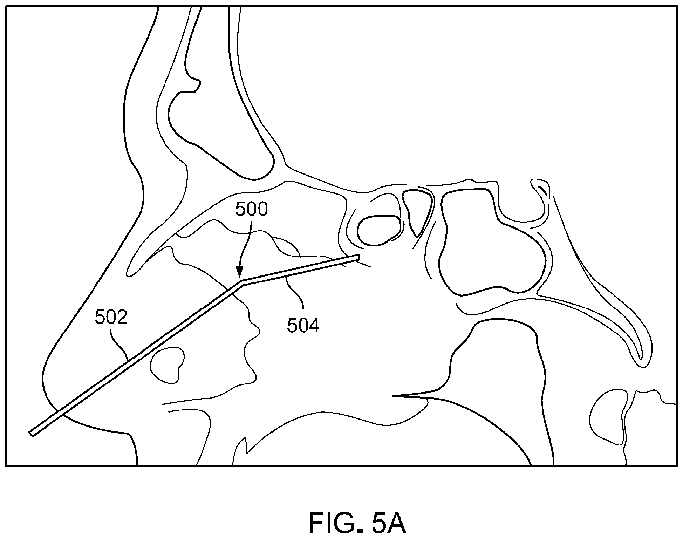

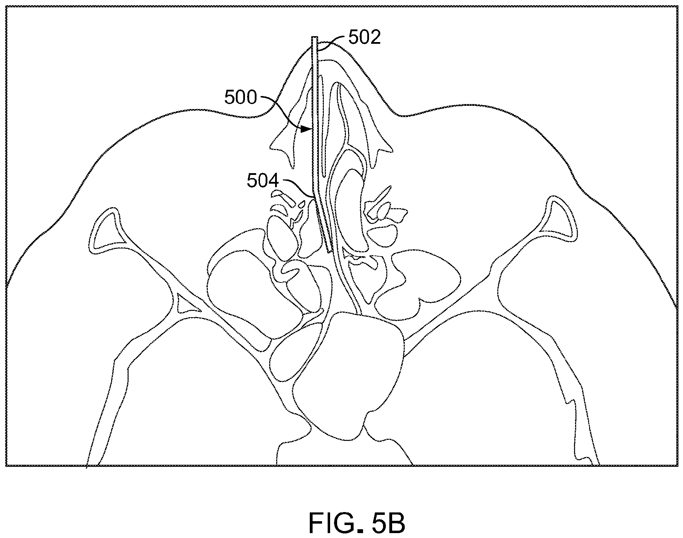

FIGS. 5A-5B illustrate a drug delivery device according to particular implementations. In FIG. 5A, the drug delivery device 500 is positioned in the submucosal space for depot delivery of a therapeutic agent in a manner similar to device 100. As shown in FIG. 5A, the branch portion 504 of the device 500 is substantially parallel to the olfactory bulb and positioned in the submucosal space. As demonstrated in FIG. 5B, which provides a top view of the drug delivery device 500 in the submucosal space, in certain implementations the drug delivery device 500 can be angled about a second axis. Accordingly, the device is also configured for extending inwardly through the nasal cavity to reach the submucosal space.

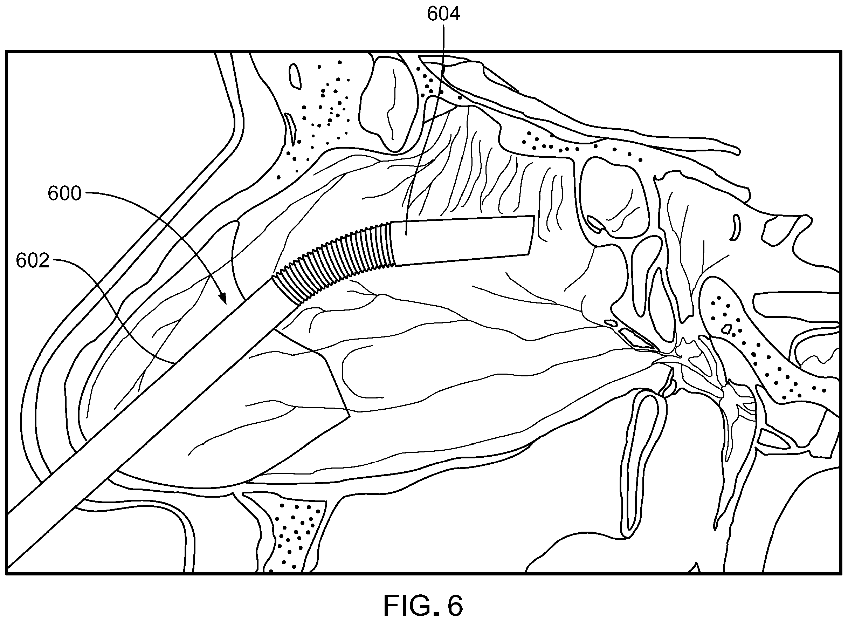

FIG 6 shows a side sectional view of another drug delivery device according to certain implementations. As illustrated in FIG. 6, a drug delivery device 600 can be configured to flex and thereby change the degree of the obtuse angle between a branch portion 604 and a stem portion 602 of the cannulated drug delivery device 600. The flexibility can be provided about more than one axis in particular implementations.

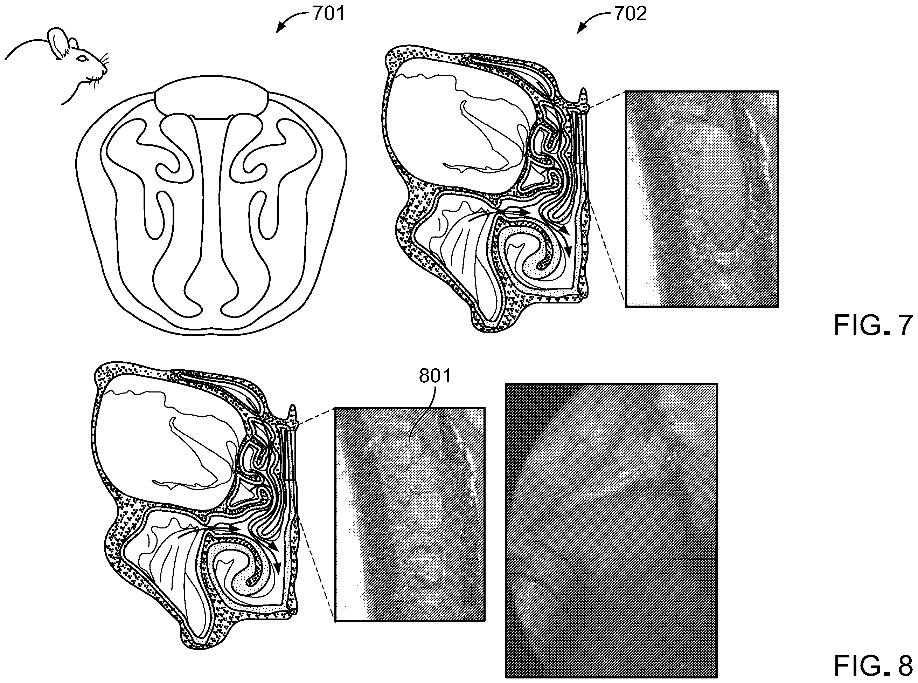

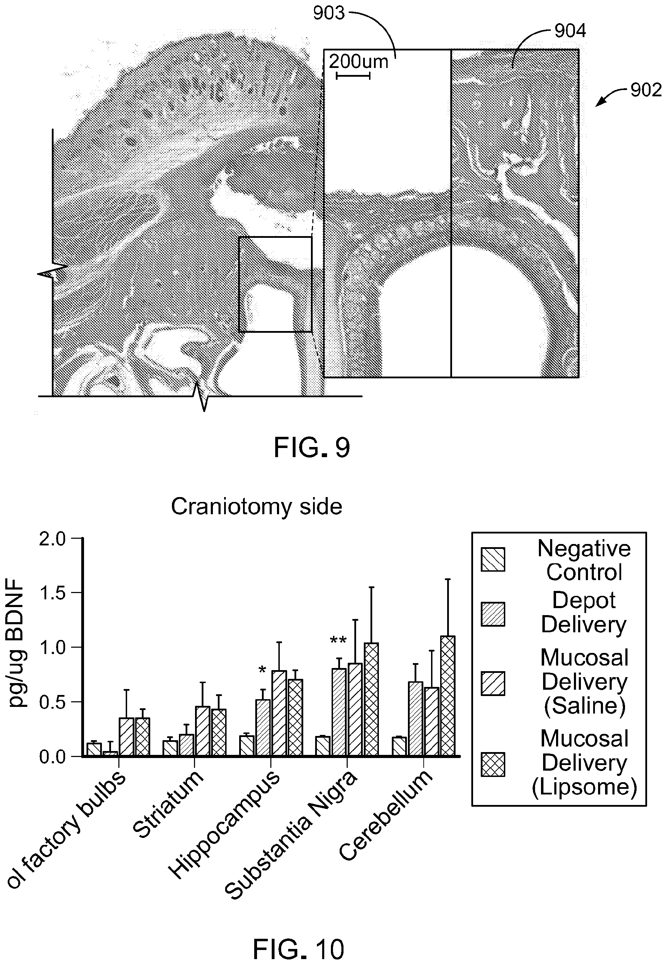

FIG. 7 illustrates a cross sectional illustration of rat model of a depot. In the rat model 701, the depot is delivered where the dorsal nasal bone is removed (as shown in FIG. 9) from the rat to expose the mucoperichondrium of the nasal and olfactory rodent mucosa. The depot is then applied to this surface, which is anatomically identical to the submucosal space in the human model 702.

FIG. 8 shows an endoscopic view of the olfactory submucosal space with an olfactory nerve trunk 801 seen extending from the intracranial space into the submucosal space. This space represents the potential space where the depot will be dosed.

FIG. 9 is a cross sectional histological view (H&E) of the rat model 701 depicted in FIG. 7 at low and high magnification. The split expanded box 902 represents the post surgical view 903 and pre-surgical view 904 (bar = 250 microns) before and after dorsal nasal bone removal.

FIG. 10 shows a comparison of ipsi-lateral BDNF upregulation (e.g. craniotomy side) following BDNF AntagoNAT (0. 15mg/kg) delivery via a mucosal graft in saline or liposome, versus minimally invasive nasal depot (MIND), versus saline control in various brain subregions. The depot approach results in significant BDNF upregulation relative to control in hippocampus and substantia nigra relative to negative control.

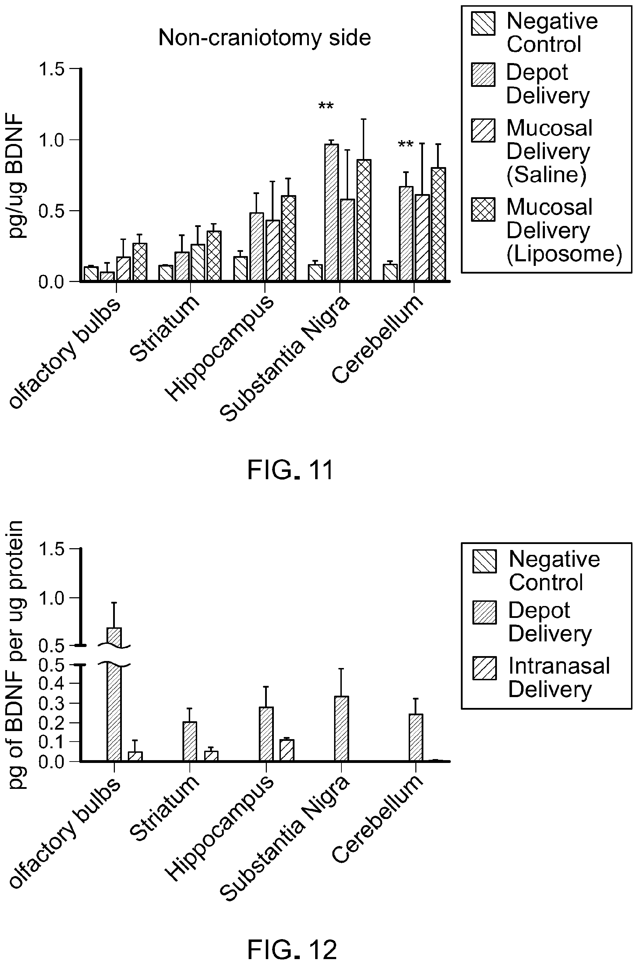

FIG. 11 shows a comparison of contra-lateral BDNF upregulation (e.g. non-craniotomy side) following BDNF AntagoNAT (0. 15mg/kg) delivery via a mucosal graft in saline or liposome, versus minimally invasive nasal depot (MIND), versus saline control in various brain subregions. The depot approach results in significant BDNF upregulation relative to control in substantia nigra and cerebellum relative to negative control.

FIG. 12 shows a comparison of BDNF AntagoNAT (0. 15mg/kg, 60micL) distribution in the rat brain following depot delivery versus traditional nasal irrigation and negative saline control demonstrating increased distribution in all regions examined using the minimally invasive nasal depot (MIND) technique.

### OTHER EMBODIMENTS

While this specification contains many specific implementation details, these should not be construed as limitations on the scope of any inventions or of what may be claimed, but rather as descriptions of features specific to particular implementations of particular inventions. Certain features that are described in this specification in the context of separate implementations can also be implemented in combination in a single implementation. Conversely, various features that are described in the context of a single implementation can also be implemented in multiple implementations separately or in any suitable sub combination. Moreover, although features may be described above as acting in certain combinations and even initially claimed as such, one or more features from a claimed combination can in some cases be excised from the combination, and the claimed combination may be directed to a sub combination or variation of a sub combination.

While this specification contains many specific implementation details, these should not be construed as limitations on the scope of any inventions or of what may be claimed, but rather as descriptions of features specific to particular implementations of particular inventions. Certain features that are described in this specification in the context of separate implementations can also be implemented in combination in a single implementation. Conversely, various features that are described in the context of a single implementation can also be implemented in multiple implementations separately or in any suitable sub combination. Moreover, although features may be described above as acting in certain combinations and even initially claimed as such, one or more features from a claimed combination can in some cases be excised from the combination, and the claimed combination may be directed to a sub combination or variation of a sub combination.

The orientation of various elements may differ according to other exemplary implementations, and such variations are encompassed by the present disclosure. Features of the disclosed implementations can be incorporated into other disclosed implementations.

While various inventive implementations have been described and illustrated herein, a variety of other means and/or structures for performing the function and/or obtaining the results and/or one or more of the advantages described herein are, and each of such variations and/or modifications is, deemed to be within the scope of the implementations described herein. The foregoing implementations are presented by way of example only and that, within the scope of the appended claims, inventive implementations may be practiced otherwise than as specifically described and claimed. Implementations of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

Also, the technology described herein may be embodied as an example method, of which at least one example has been provided. The acts performed as part of the method may be ordered in any suitable way. Accordingly, implementations may be constructed in which acts are performed in an order different than illustrated, which may include performing some acts simultaneously, even though shown as sequential acts in illustrative implementations.

The claims should not be read as limited to the described order or elements unless stated to that effect. It should be understood that various changes in form and detail may be made by one of ordinary skill in the art without departing from the scope of the appended claims. The invention is defined by the following claims.

## Claims

1. A nasal drug delivery device comprising:
a catheter component (100) comprising a stem portion (102) and a branch portion (104), the catheter component (100) comprising a pre-defined bend such that the branch portion (104) is angled with respect to the stem portion (102) at an obtuse angle, the branch portion (104) comprising a dissecting head (106) positioned at the tip of the branch portion (104), the dissecting head (106) comprising a flattened disc geometry and at least one delivery port (201) positioned in the dissecting head (106);
a dilation balloon (108) positioned on the branch portion (104) between the pre-defined bend and the dissecting head (106), the dilation balloon (108) configured to expand around at least a portion of a cylindrical wall of the branch portion (104);
a drug delivery reservoir (118) fluidly coupled to the at least one delivery port (201) through the branch portion (104) and through at least a portion of the stem portion (102); and
a handle portion (116) coupled to the stem portion (102).

2. The nasal drug delivery device of claim 1, wherein the branch portion (104) is angled with respect to the stem portion (102) at an obtuse angle about two axes.

3. The nasal drug delivery device of claim 1 or claim 2, further comprising an outer sheath (110) positioned between the pre-defined bend and the dilation balloon (108).

4. The nasal drug delivery device of claim 3, wherein the branch portion (104) is configured to retract into and extend from the outer sheath (110) at the obtuse angle, or, wherein at least a portion of the branch portion (104) is configured to retract into and extend from the outer sheath (110) at the obtuse angle; and/or, wherein the dilation balloon (108) is configured to retract into and extend from the outer sheath (110) at the obtuse angle with the branch portion (104), and/or, wherein the branch portion (104) is configured to extend 2-3 cm pass the outer sheath (110).

5. The nasal drug delivery device of any one of claims 1-4, comprising an endoscope (112) positioned adjacent to the pre-defined bend so as to have a field of view of the dissecting head (106), preferably, wherein the endoscope (112) comprises a camera.

6. The nasal drug delivery device of any one of claims 1-5, wherein the dissecting head (106) comprises a cannula having a diameter in the range of 0.25 mm and 3 mm.

7. The nasal drug delivery device of any one of claims 1-6, further comprising a pump communicably coupled to the drug delivery reservoir (118) and configured to move a drug from the drug delivery reservoir (118) to the at least one delivery port (201), preferably, wherein the pump comprises a plunger assembly.

8. The nasal drug delivery device of any one of claims 1-7, wherein the obtuse angle is in the range of 155-170 degrees.

9. The nasal drug delivery device of any one of claims 1-8, wherein the branch portion (104) has a length in the range of 10-20cm.

10. The nasal drug delivery device of any one of claims 1-9, wherein the dilation balloon (108) has length in the range of 1-4 cm, and/or, wherein the dilation balloon (108) has a diameter in the range of 0.1-3 mm.

11. The nasal drug delivery device of any one of claims 1-10, wherein the branch portion (104) extends upstream and downstream of the dilation balloon (108) such that the dilation balloon (108) is positioned between the dissecting head (106) and a section of the branch portion (104) that is narrower than dilation balloon (108) in an inflated state, whereby an entry to a depot upon retraction of the branch portion (104) is narrower than the depot itself.

12. The nasal drug delivery device of any one of claims 1-11, wherein the drug delivery reservoir (118) contains a therapeutically effective amount of a drug for treating a central nervous system disorder.

13. The nasal drug delivery device of any one of claims 1-12, wherein the dissecting head (106) is configured to create an opening having a cross sectional area that is smaller than a cross sectional area of the dilation balloon (108) when inflated.

## Patentansprüche

1. Vorrichtung zur nasalen Verabreichung von Arzneimitteln, umfassend:
Eine Katheter-Komponente (100), die einen Stammabschnitt (102) und einen Abzweigabschnitt (104) umfasst, wobei die Katheter-Komponente (100) eine vordefinierte Biegung derart umfasst, dass der Abzweigabschnitt (104) in Bezug auf den Stammabschnitt (102) in einem stumpfen Winkel abgewinkelt ist, der Abzweigabschnitt (104) einen an der Spitze des Abzweigabschnitts (104) positionierten Sezierkopf (106) umfasst, der Sezierkopf (106) die Geometrie einer abgeflachten Scheibe und zumindest eine Öffnung (201) zur Verabreichung umfasst, die im Sezierkopf (106) positioniert ist;
einen Erweiterungsballon (108), der am Abzweigabschnitt (104) zwischen der vordefinierten Biegung und dem Sezierkopf (106) positioniert ist, wobei der Erweiterungsballon (108) konfiguriert ist, sich herum um zumindest einen Abschnitt einer zylindrischen Wand des Abzweigabschnitts (104) zu erweitern;
ein Reservoir (118) für die Arzneimittelverabreichung, das flüssig an die zumindest eine Öffnung (201) zur Verabreichung durch den Abzweigabschnitt (104) und durch zumindest einen Abschnitt des Stammabschnitts (102) gekoppelt ist; und einen an den Stammabschnitt (102) gekoppelten Griffabschnitt (116).

2. Vorrichtung zur nasalen Verabreichung von Arzneimitteln nach Anspruch 1, wobei der Abzweigabschnitt (104) in Bezug auf den Stammabschnitt (102) in einem stumpfen Winkel um zwei Achsen abgewinkelt ist.

3. Vorrichtung zur nasalen Verabreichung von Arzneimitteln nach Anspruch 1 oder Anspruch 2, die weiter eine Außenhülse (110) umfasst, die zwischen der vordefinierten Biegung und dem Erweiterungsballon (108) positioniert ist.

4. Vorrichtung zur nasalen Verabreichung von Arzneimitteln nach Anspruch 3, wobei der Abzweigabschnitt (104) konfiguriert ist, sich am stumpfen Winkel in die Außenhülse (110) zurückzuziehen und sich aus dieser zu erstrecken oder, wobei zumindest ein Abschnitt des Abzweigabschnitts (104) konfiguriert ist, sich am stumpfen Winkel in die Außenhülse (110) zurückzuziehen und sich aus dieser zu erstrecken; und/oder, wobei der Erweiterungsballon (108) konfiguriert ist, sich am stumpfen Winkel mit dem Abzweigabschnitt (104) in die Außenhülse (110) zurückzuziehen und sich aus dieser zu erstrecken und/oder, wobei der Abzweigabschnitt (104) so konfiguriert ist, dass er 2-3 cm über die Außenhülse (110) hinausragt.

5. Vorrichtung zur nasalen Verabreichung von Arzneimitteln nach irgendeinem der Ansprüche 1-4, die ein Endoskop (112) umfasst, das angrenzend an die vordefinierte Biegung so positioniert ist, dass es ein Sichtfeld auf den Sezierkopf (106) hat, vorzugsweise, wobei das Endoskop (112) eine Kamera umfasst.

6. Vorrichtung zur nasalen Verabreichung von Arzneimitteln nach irgendeinem der Ansprüche 1-5, wobei der Sezierkopf (106) eine Kanüle mit einem Durchmesser im Bereich von 0,25 mm und 3 mm aufweist.

7. Vorrichtung zur nasalen Verabreichung von Arzneimitteln nach irgendeinem der Ansprüche 1-6, die weiter eine Pumpe umfasst, die kommunikativ an das Reservoir (118) für die Arzneimittelverabreichung gekoppelt und konfiguriert ist, ein Arzneimittel aus dem Reservoir (118) für die Arzneimittelverabreichung zur zumindest einen Öffnung (201) zur Verabreichung zu pumpen, vorzugsweise, wobei die Pumpe eine Kolbenbaugruppe umfasst.

8. Vorrichtung zur nasalen Verabreichung von Arzneimitteln nach irgendeinem der Ansprüche 1-7, wobei der stumpfe Winkel im Bereich von 155-170 Grad liegt.

9. Vorrichtung zur nasalen Verabreichung von Arzneimitteln nach irgendeinem der Ansprüche 1-8, wobei der Abzweigabschnitt (104) eine Länge im Bereich von 10-20 cm aufweist.

10. Vorrichtung zur nasalen Verabreichung von Arzneimitteln nach irgendeinem der Ansprüche 1-9, wobei der Erweiterungsballon (108) eine Länge im Bereich 1-4 cm aufweist und/oder, wobei der Erweiterungsballon (108) einen Durchmesser im Bereich von 0,1-3 mm aufweist.

11. Vorrichtung zur nasalen Verabreichung von Arzneimitteln nach irgendeinem der Ansprüche 1-10, wobei sich der Abzweigabschnitt (104) so stromaufwärts und stromabwärts des Erweiterungsballons (108) erstreckt, dass er zwischen dem Sezierkopf (106) und einem Teil des Abzweigabschnitts (104) positioniert ist, der schmaler als der Erweiterungsballon (108) in einem aufgeblasenen Zustand ist, wobei ein Eingang zu einem Depot beim Zurückziehen des Abzweigabschnitts (104) schmaler als das Depot selbst ist.

12. Vorrichtung zur nasalen Verabreichung von Arzneimitteln nach irgendeinem der Ansprüche 1-11, wobei das Reservoir (118) für die Verabreichung des Arzneimittels eine therapeutisch wirksame Menge eines Arzneimittels zur Behandlung einer Erkrankung des zentralen Nervensystems enthält.

13. Vorrichtung zur nasalen Verabreichung von Arzneimitteln nach irgendeinem der Ansprüche 1-12, wobei der Sezierkopf (106) so konfiguriert ist, dass er eine Öffnung mit einer Querschnittsfläche schafft, die kleiner als eine Querschnittsfläche des Erweiterungsballons (108), wenn aufgeblasen, ist.

## Revendications

1. Dispositif d'administration nasale de médicament, comprenant :
un élément cathéter (100) comprenant une partie tige (102) et une partie branche (104), l'élément cathéter (100) comprenant une courbure prédéfinie de telle sorte que la partie branche (104) soit inclinée par rapport à la partie tige (102) selon un angle obtus, la partie branche (104) comprenant une tête de dissection (106) positionnée au niveau de l'extrémité de la partie branche (104), la tête de dissection (106) comprenant une géométrie de disque aplati et au moins un orifice d'administration (201) positionné dans la tête de dissection (106) ;
un ballonnet de dilatation (108) positionné sur la partie branche (104) entre la courbure prédéfinie et la tête de dissection (106), le ballonnet de dilatation (108) étant configuré pour se dilater autour d'au moins une partie d'une paroi cylindrique de la partie branche (104) ;
un réservoir d'administration de médicament (118) accouplé fluidiquement à l'au moins un orifice d'administration (201) par l'intermédiaire de la partie branche (104) et par l'intermédiaire d'au moins une partie de la partie tige (102) ; et
une partie poignée (116) accouplée à la partie tige (102).

2. Dispositif d'administration nasale de médicament selon la revendication 1, dans lequel la partie branche (104) est inclinée par rapport à la partie tige (102) selon un angle obtus autour de deux axes.

3. Dispositif d'administration nasale de médicament selon la revendication 1 ou 2, comprenant en outre une gaine extérieure (110) positionnée entre la courbure prédéfinie et le ballonnet de dilatation (108).

4. Dispositif d'administration nasale de médicament selon la revendication 3, dans lequel la partie branche (104) est configurée pour se rétracter dans la gaine extérieure (110) et s'étendre à partir de celle-ci selon l'angle obtus, ou dans lequel au moins une partie de la partie branche (104) est configurée pour se rétracter dans la gaine extérieure (110) et s'étendre à partir de celle-ci selon l'angle obtus ; et/ou dans lequel le ballonnet de dilatation (108) est configuré pour se rétracter dans la gaine extérieure (110) et s'étendre à partir de celle-ci selon l'angle obtus avec la partie branche (104), et/ou dans lequel la partie branche (104) est configurée pour s'étendre sur 2 à 3 cm au-delà de la gaine extérieure (110).

5. Dispositif d'administration nasale de médicament selon l'une quelconque des revendications 1 à 4, comprenant un endoscope (112) positionné à proximité de la courbure prédéfinie afin de permettre l'obtention d'un champ de vision de la tête de dissection (106), l'endoscope (112) comprenant de préférence une caméra.

6. Dispositif d'administration nasale de médicament selon l'une quelconque des revendications 1 à 5, dans lequel la tête de dissection (106) comprend une canule ayant un diamètre de l'ordre de 0,25 mm à 3 mm.

7. Dispositif d'administration nasale de médicament selon l'une quelconque des revendications 1 à 6, comprenant en outre une pompe accouplée en communication au réservoir d'administration de médicament (118) et configurée pour déplacer un médicament du réservoir d'administration de médicament (118) vers l'au moins un orifice d'administration (201), la pompe comprenant de préférence un ensemble piston.

8. Dispositif d'administration nasale de médicament selon l'une quelconque des revendications 1 à 7, dans lequel l'angle obtus est de l'ordre de 155 à 170 degrés.

9. Dispositif d'administration nasale de médicament selon l'une quelconque des revendications 1 à 8, dans lequel la partie branche (104) a une longueur de l'ordre de 10 à 20 cm.

10. Dispositif d'administration nasale de médicament selon l'une quelconque des revendications 1 à 9, dans lequel le ballonnet de dilatation (108) a une longueur de l'ordre de 1 à 4 cm, et/ou dans lequel le ballonnet de dilatation (108) a un diamètre de l'ordre de 0,1 à 3 mm.

11. Dispositif d'administration nasale de médicament selon l'une quelconque des revendications 1 à 10, dans lequel la partie branche (104) s'étend en amont et en aval du ballonnet de dilatation (108) de telle sorte que le ballonnet de dilatation (108) soit positionné entre la tête de dissection (106) et une section de la partie branche (104) qui est plus étroite que le ballonnet de dilatation (108) dans un état gonflé, de telle sorte qu'une entrée dans un dépôt lors de la rétraction de la partie branche (104) soit plus étroite que le dépôt lui-même.

12. Dispositif d'administration nasale de médicament selon l'une quelconque des revendications 1 à 11, dans lequel le réservoir d'administration de médicament (118) contient une quantité thérapeutiquement efficace d'un médicament destiné à traiter un trouble du système nerveux central.

13. Dispositif d'administration nasale de médicament selon l'une quelconque des revendications 1 à 12, dans lequel la tête de dissection (106) est configurée pour créer une ouverture ayant une section transversale qui est inférieure à une section transversale du ballonnet de dilatation (108) lorsqu'il est gonflé.
